(19) Europäisches Patentamt
European Patent Office
Office européen des brevets

(11) Publication number: **0 047 345**
**B1**

(12) **EUROPEAN PATENT SPECIFICATION**

(45) Date of publication of patent specification: **07.12.83**

(51) Int. Cl.³: **C 07 C 29/15**, C 07 C 31/04

(21) Application number: **80302992.5**

(22) Date of filing: **28.08.80**

(54) **Process for the synthesis of methanol from hydrogen and the oxides of carbon using catalysts.**

(43) Date of publication of application:
**17.03.82 Bulletin 82/11**

(45) Publication of the grant of the patent:
**07.12.83 Bulletin 83/49**

(84) Designated Contracting States:
**BE DE FR GB NL**

(56) References cited:
**DE - B - 1 667 345**
**FR - A - 2 181 002**
**GB - A - 1 302 726**
**GB - A - 1 364 096**
**US - A - 3 254 967**

(73) Proprietor: **Wentworth, Theodore Oscar**
**P O Box 3279**
**Cincinnati Ohio 45201 (US)**

(72) Inventor: **Stiles, Alvin Barber**
**1301 Grayson Road Welshire**
**Wilmington Delaware 19803 (US)**
Inventor: **Wentworth, Theodore Oscar**
**Post Office Box 1042**
**Carefree Arizona 85377 (US)**

(74) Representative: **Oliver, Roy Edward et al,**
**POLLAK MERCER & TENCH High Holborn House**
**52-54 High Holborn**
**London WC1V 6RY (GB)**

EP 0 047 345 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for the synthesis of methanol from hydrogen and the oxides of carbon using catalysts

This invention relates to an improvement in the process for the synthesis of methanol wherein an original gaseous mixture containing hydrogen and at least one of the oxides of carbon is passed under conditions of elevated temperature and pressure into contact with a plurality of catalysts in succession.

When synthetic methanol is produced by passing a synthesis gas comprising a mixture of hydrogen and one or more carbon oxides (carbon monoxide, carbon dioxide, or a mixture of carbon monoxide and carbon dioxide) into contact with a catalyst under conditions of elevated temperature and pressure, the particular reaction temperature chosen is related to the type of catalyst used. Certain catalysts will operate effectively at as low as 220°C., whereas others require temperatures above 315°C. to operate satisfactorily. The lower temperatures favor methanol in the equilibrium according to the reaction:

$$(1)\ CO + 2H_2 \rightarrow CH_3OH$$

However, the lower temperatures are less favorable for the equilibrium toward carbon monoxide according to the reverse shift reaction which is necessary to produce methanol from carbon dioxide:

$$(2)\ CO_2 + H_2 \rightarrow CO + H_2O$$

In view of these two considerations of chemical equilibrium, it is an object of this invention to utilize the characteristics of more than one catalyst and operate over a wide range of temperature conditions favorable for both the above reactions to occur.

It will be noted that the two preceding reactions are not the only ones which occur when the process is carried out. Thus, in the case where the carbon oxide in the synthesis gas or the make-up feed is all carbon monoxide, the gas contacting the catalyst will react to form small amounts of byproducts, such as dimethyl ether and higher alcohols, with the formation of water vapor. For instance, in the case of dimethyl ether,

$$(3)\ 2CO + 4H_2 \rightarrow CH_3OCH_3 + H_2O$$

and in the case of any of the four butyl alcohols,

$$(4)\ 4CO + 8H_2 \rightarrow C_4H_9OH + 3H_2O$$

The water vapor produced in (3) or (4) will react by the reverse of reaction (2) to form some carbon dioxide to an extent limited by the chemical equilibrium of the reaction mixture.

The reaction of hydrogen and one or more carbon oxides to produce methanol produces considerable heat, resulting in an increase in the temperature of the stream of gas being processed. For efficient operation, however, it is essential that the reaction temperature be rather closely controlled. This has been accomplished in the past by using a direct cold gas quench. Note U.S. Patent No. 3,254,967 to T.O. Wentworth and U.S. Patent No. 3,480,407 to T.O. Wentworth and C.G. Anderson. Such operations have been carried out using a catalyst having a single composition.

It is to be noted that higher pressures favor the equilibrium of reaction (1) and higher temperatures favor reaction (2).

In addition to the chemical equilibrium considerations, the catalysts selected in the practice of this invention are arranged in a particular sequence or assemblage, which will minimize the inlet temperature of the initial gaseous mixture to the first bed, thereby requiring less heat-exchange to heat the gases to the proper temperature for initiation of the reaction.

Since the gases pass over catalysts at successively higher temperatures, then are cooled, and then pass into at least one or more catalyst beds at a lower temperature, the following beneficial results are achieved:

1. The $CO_2$ in the synthesis gas is reverse-shifted to CO under equilibrium conditions at the highest temperature in the last stage of the first reaction sequence. As stated above, this reverse-shift is favored because: (a) same CO has already been converted to methanol; (b) the reaction temperature is higher; and finally because (c) a catalyst specifically capable of promoting the shift of $CO_2$ plus $H_2$ to CO plus $H_2O$ is present in this bed. The gases with increased CO content and reduced $CO_2$ content are then cooled to a lower temperature at which the methanol equilibrium is favored and a catalyst specific for methanol synthesis is employed in this first bed following the initial assemblage of beds.

It is also to be noted that reaction (1) is exothermal whereas reaction (2) is endothermal, so that there is a balancing of temperature effects in the higher temperature beds, tending to make the reactor more nearly isothermal, which also favors efficiency of operation, i.e., minimizes formation of byproducts.

By the use of this invention, capital costs and maintenance costs are decreased for a plant of a given methanol production capacity because of the significant decrease in the quantity of gas circulated for each unit of methanol produced. The sizes, and therefore the costs, of the circulation compressor,

piping, heat-exchangers, and liquid-gas separator in the synthesis "loop" are substantially reduced, in comparison with existing processes with their higher circulating rates.

Detailed Description

The improvements of the present invention and operating costs are the result of two changes in conventional methanol synthesis processes. First, in the present process, indirect heat-exchange is employed for the purpose of recovering heat of reaction. Second, the process utilizes two or more different catalyst compositions capable of operating within different, specified temperature ranges. These catalysts are known in the art. Note, Emmett, *Catalysis*, Volume III (1955) pages 364—377; and Kirk-Othmer, *Encylopedia of Chemical Technology*, Second Edition, Volume 13 (1967) page 379.

The process of this invention is one in which the synthesis gas is initially passed through a first assemblage of two or more beds arranged in series containing different catalysts. These beds of catalyst, in the direction of flow of the gas, are of such composition as to function most effectively at increasingly higher temperatures. The first bed of catalyst is one which produces methanol at a low initial reaction temperature, thereby minimizing the amount of heat-exchange required to heat the inlet gas to a temperature appropriate for the methanol synthesis reaction to initiate and also taking advantage of the more favorable equilibrium of equation (1) at lower temperatures. The last bed of catalyst in the first assemblage or sequence of catalyst beds in one which operates at a high temperature thereby taking advantage of the reverse-shift reaction of equation (2). Preferably, the gas entering the first bed is at a temperature within the range from 200°C. to 260°C. and is at a pressure of from 70 kg/sq.cm. to 527 kg/sq.cm. Preferably also, the gas exiting the last bed in the first assemblage is at a temperature within the range from 370°C. to 400°C. and is also at a pressure of from 70 kg/sq.cm. to 527 kg/sq.cm. Throughout this specification, "kg/sq.cm." means kilograms per square centimeter gage pressure.

The gaseous stream undergoing reaction, after exiting the last bed in the series of the first assemblage is cooled by indirect heat-exchange and is then introduced into a second assemblage of one or more catalyst beds. If there is one bed, the temperature remains relatively low. By operating in this manner, advantage is taken of the favorable equilibrium of equation (1) at lower temperatures. Carbon monoxide, including that produced in, and exiting from, the last bed in the first assemblage, is thereby converted to an increased extent to methanol. If more than one bed of catalyst is present in the second assemblage after the indirect heat-exchange step, the beds are arranged in series, and the beds contain catalysts of such compositions as are designed to function at increasingly higher temperatures in the direction of flow of the reaction gas stream. The temperature of the reaction mixture leaving the last bed in the second assemblage will be in the range from the temperature of the gas introduced into the first bed of catalyst in the first assemblage to the temperature of the gas exiting the last bed of the first assemblage, which is that bed immediately preceding the heat-exchange step. Preferably, the temperature of the gas entering the first bed in the second assemblage will be within the range from 200°C. to 260°C. and the temperature of the gas leaving the last bed of this second assemblage will be within the range from 285°C. to 345°C. or even lower than 285°C.

The process of this invention also includes one in which the gas leaving the last bed in the second assemblage is cooled by indirect heat-exchange and is then introduced into a third assemblage consisting of at least one further bed of catalyst. In this case, an advantage is obtained from the favorable equilibrium of equation (1) at lower temperatures. If more than one bed of catalyst is used after the second heat-exchange step, the beds are arranged in series, and the beds contain catalysts having compositions designed to function at increasingly higher temperatures in the direction of flow of the reaction stream. The temperature of the reaction mixture leaving the last bed of this third assemblage after the second heat-exchange step will range between the temperature of the gas introduced into the first bed of catalyst of the first assemblage and the temperature of the gas exiting the last bed in the second assemblage. Preferably, the temperature of the gas entering the first bed after the second heat-exchange step will be within the range from 200°C. to 260°C. and the temperature of the gas leaving the last bed after the second heat-exchange step will be within the range from 260°C. to 285°C.

In one preferred embodiment, the process of this invention is one in which the synthesis gas is passed through three (first, second and third) beds of catalyst comprising a first assemblage, is then cooled by indirect heat-exchange, and is then passed through two more (fourth and fifth) beds of catalyst comprising a second assemblage.

The first bed of catalyst is initially contacted at a temperature within the range from 200°C. to 260°C. The first bed contains a catalyst which functions over temperatures within the range from 200°C. to 285°C. to produce methanol from hydrogen and carbon monoxide. The second bed contains a catalyst which functions over temperatures within the range from 260°C. to 335°C. to produce methanol from hydrogen and carbon monoxide. As carbon monoxide is converted to methanol in the first and second beds, the reverse shift reaction of equation (2), particularly if a suitable catalyst is present, converts carbon dioxide to carbon monoxide, making more carbon monoxide available to react to form methanol.

Continuing this embodiment, the third bed is composed of catalyst which functions over temperatures within the range from 315°C. to 400°C. to produce methanol from hydrogen and carbon

monoxide. These higher temperatures promote more reverse shift to produce more carbon monoxide from carbon dioxide. Catalysts suitable for each of the above services are more fully described hereinafter.

Still continuing this more specific embodiment, the effluent from the third bed of catalyst is passed in indirect heat-exchange relationship with a cooling medium (suitably, boiler feed water) whereby the temperature of the effluent is reduced to a temperature within the range from 200°C. to 260°C. If desired, a part of the purified and preheated synthesis gas enters the fourth bed of catalyst within passing through the first assemblage of the three beds of catalysts.

Still continuing this more specific embodiment, the effluent from the heat-exchanger is introduced into a first bed of catalyst in a second assemblage of catalyst beds which contains catalyst that functions over temperatures within the range from 200°C. to 285°C. to produce methanol from hydrogen and carbon monoxide. This catalyst is preferably the same as that employed in the first bed of the first assemblage of reactor beds.

Finally, with respect to this more specific embodiment, the effluent from the first bed of catalyst of the second assemblage into a second bed containing catalyst which functions over temperatures within the range of 260°C. to 345°C. to produce methanol from hydrogen and carbon monoxide. This catalyst is preferably the same as that employed in the second bed of the first assemblage of reaction beds.

The temperature of the gases exiting this second bed is from 285°C. to 345°C. and is much lower than the temperature of gases exiting the third bed (370°C. to 400°C.) in the first assemblage of converter beds and prior to heat exchange and gas cooling. The concentration of the methanol in the gas stream exiting this last bed can reach 7.5/mol per cent at 35 per cent of methanol equilibrium.

Description of Catalysts

The invention preferably requires the use of at least three general types or classes of catalysts, all known to the art. For the first time this knowledge is being applied to a new method for methanol synthesis. These catalysts are hereinafter, designated as C—1, C—2 and C—3 (also at times hereinafter, respectively, as $C_1$ ,$C_2$, and $C_3$.) They are to be considered not as individual catalysts, but as classes of catalysts which will perform as required in each service and which will differ from each other primarily in the temperature range in which each is most suitable for use. There is some over-lapping of temperatures in which each class of catalysts is capable of functioning, but despite this over-lap each class should be considered as distinct.

In general, all of the catalysts must be capable of performing the following functions: The catalyst class must initiate the reaction at the lowest temperature of the range indicated. For example, for Class C—1 the catalyst must initiate the reaction at 175°C. and be stable and effective to temperatures as high as 280°C. Class C—2 has the same requirements but for a temperature range of 245°C. to 330°C., whereas class C—3 has the range of 300°C. to above 395°C.

All catalysts in all classes should have the following characteristics : They should be tolerant to poisons such as sulfur and halide, or they should be so adsorptive that they will remove the poisons in the upstream portion of the catalyst bed and thus protect the downstream portion for normal action. Most catalysts containing copper and zinc are highly adsorptive of these poisons, whereas these metals can be made tolerant to these same poisons by converting the copper and zinc to such "salts" as chromites ($CuCr_2O_4$), for example, or by the incorporation of such acidic or amphoteric oxides as ceria, mixed rare earth oxides, alumina, titania, zirconia, lanthana, thoria, and combinations of oxides, such as magnesium aluminate spinels and the like. The latter have the further effect of introducing an incipiently isometric nucleus into the catalytic structure. These oxides can be incorporated by co-precipitation of nitrate salts using ammonium hydroxide, ammonium bicarbonate, ammonium carbonate, sodium carbonate or bicarbonate, oxalate, and other alkali salts of these precipitants. It is preferable that the oxides named above be finely divided, and co-precipitation is therefore the preferred method for introducing them, however, some beneficial effect can be obtained by intensively and intimately grinding together the finely divided oxides, for example, copper oxide, zinc oxide and ceria. Other methods will be evident to one skilled in the art.

The catalysts should also be resistant to exposure to the temperatures employed. The catalysts should be capable of sustaining their performances for months or even years, so that the slow growth in crystal size which attends some forms of catalyst deactivation is forestalled, even at temperatures slightly above operating range, because there may be operating upsets which cause temperature excursions above the normal operating range. The agents which make the catalysts resistant to the temperature and time effect are essentially the same as those added above to attain poison resistance. Thus, it is desirable to choose a component of the catalyst which will have the dual effect of imparting both poison and temperature resistance. Essentially all of the above mentioned additives do so, but of particular effectiveness are chromia, ceria, mixed rare earth oxides and zirconia. This is not to say that some benefit is not obtained with the others mentioned such as alumina, magnesium aluminate spinel and titania.

The metals comprisings the catalysts themselves are as follows:

C—1 is comprised primarily of copper and zinc as the oxides with the copper being in the atom ratio of 1 to more than 6 to each zinc (i.e., 1:1 Cu to Zn to 6:1 Cu to Zn). These can be derived from the

nitrate salts and precipitated with the precipitants already described and stabilized with the stabilizers described above. The stabilizers can be in the proportion of from one per cent to 60 per cent, or even greater, based on the total weight of copper, zinc and stabilizer oxides.

C—2 is also comprised of copper and zinc as the oxides, but with an atom ratio of copper to zinc of 1 to 0.25. This C—2 catalyst can also differ from C—1 in that it can include some manganese (particularly if higher alcohols are desired), and can also include a higher quantity of thermal stabilizers. Because of the higher temperature of operation, the thermal stabilizer content is preferably in a range approaching 20% to as high as 65%, or even higher, based on the total weight of copper, zinc and stabilizer oxides.

C—3 is ordinarily zinc chromite, but may contain copper oxide as such, or as chromite. This catalyst group may also contain manganese, particularly if higher alcohols are desired as a significant fraction of the product. The further requirement of this catalyst, as explained in the specification above, is that it be effective in the conversion of $CO_2$ and $H_2$ to CO and water vapor. This is achieved either by the incorporation into the catalyst composition, or as a component of a mixed charge of catalyst, of such very effective catalysts as a copper oxide — zinc oxide catalyst containing the copper in an atom ratio of 1:2 to the zinc. This catalyst can thus conveniently be copper-zinc chromite having a ratio 1:2:3 of Cu:Zn:Cr and can be made by precipitation from the nitrates of copper and zinc in the presence of chromic acid. Precipitation is effected with ammonium hydroxide or other precipitants known to one skilled in the art (ammonium chromate or ammonium carbonate, for example).

All catalysts must be converted to a useable form, which ordinarily means calcining to the oxides, densification, pelleting as six mm. by six mm. Right cylinders, and finally reducing either in the methanol converter, or separately, and then charging in a completely reduced or partially reduced condition. Prior to reducing, the pellets can under some circumstances be calcined in air at temperatures approaching 485°C. to make them less susceptible to spalling and disintegration during reduction and handling.

The catalyst will preferably be distributed in the beds or trays in such a way that the same temperature rise is experienced in each bed or tray: this means that the amount of reaction or conversion (productivity) is equal in each bed. Thus, if there are five beds or trays, 20% of the total reaction is effected in each.

The quantity of catalyst may not, and very likely will not, be equal in weight or volume in each bed. For example, the first bed or converter does not need as much catalyst as the final bed, because the equilibrium or reaction driving force in the first bed where there is little methanol with be greatest, whereas in the last bed the driving force is the lowest and volume of catalyst should be greatest. Generally, the catalyst will increase in quantity from bed to bed in the first assemblage because the catalysts are intentionally designed to have equal productivity at equal pressures but at incrementally higher temperatures in the order $C_1$, $C_2$, and $C_3$. This is illustrated in the following generalized statement:

Productivity of $C_1$ at $P_1$ and $T_1$ equals
Productivity of $C_2$ at $P_1$ and $T_1$ + 30°C. equals
Productivity of $C_3$ at $P_1$ and $T_1$ + 60°C.

As the partial pressure of methanol increases, the effect is that $P_1$ is as a consequence reduced as the catalyst "sees" it or responds to it. This same effect pertains for all beds in all sequences. This is illustrated subsequently in the Examples.

Reference to Drawings

FIG. 1, 2, 3, 4 and 5 of the drawings are to be considered in connection with Examples 1, 2, 3, 4 and 5 respectively. Those skilled in the art will understand that the present invention is independent of the source of the raw material used in preparing the synthesis gas, which preferably will be composed of at least two-thirds carbon monoxide by volume, based upon the total volume of carbon monoxide and carbon dioxide. For further information concerning the preparation of synthesis gas, see Kirk-Othmer, *Encyclopedia of Chemical Technology*, Second Edition, Volume 13 (1967) pages 381—384. Examples 6, 7 and 8 describe respectively, the preparation of $C_1$, $C_2$ and $C_3$ catalyst.

Each of the figures schematically depict a methanol synthesis loop. Each Table gives the composition of the streams in the corresponding Figure. In the Tables "I" means inerts and "M/T" means pound moles per short ton of methanol. It is to be understood that methanol synthesis "loops" can vary widely in capacity, for example, from 455 metric tons per day to as large as 13,600 metric tons per day of methanol product from a single synthesis train. It is further understood that the greater the capacity the higher the optimized loop pressure will be and that pressure can range from 52.7 kg/sq.cm. to 527 kg/sq.cm. or even up to 700 kg/sq.cm.

Example I

This example describes an embodiment of the present invention in which the synthesis gas is derived from natural gas and is to be considered in conjunction with FIG. 1 of the drawings and Table I.

Referring to FIG. 1, fresh feed (also known as make-up gas) at a pressure of 295 kg/sq.cm. is

introduced into the synthesis loop through line 101 and is admixed with recycle gas flowing through line 102. The mixed stream is then pumped by circulating compressor 111 through heat-exchanger 126, wherein the temperature of the gas is raised to 232°C. To produce 0.9 metric tons of methanol, 52 brake horsepower-hours is supplied to compressor 111. Stream 103 splits, 78.35% going to stream 128 and 21.65% to stream 127.

Stream 128 then passes through three beds of catalyst $C_1$, $C_2$ and $C_3$ which are located in beds 113, 114 and 115 and comprise assemblage $A_1$. The temperature of the gas leaving bed 113 is 277°C., the temperature of the gas leaving bed 114 is 330°C., and the temperature of the gas leaving bed 115 is 376°C. These temperature increases result from the exothermicity of the reactions occurring in these catalytic reactor beds. $C_1$ is a low temperature methanol synthesis catalyst (prepared as described in Example 6) which functions at a pressure of 105 kg/cm.sq. to 527 kg/sq.cm. specifically in this Example at 281—295 kg/sq.cm. and at temperatures within the range from about 200°C. to 285°C. to produce methanol from hydrogen and a carbon oxide. $C_2$ is a medium temperature methanol synthesis catalyst (prepared as described

TABLE I

| STR | 101 | | 102 | | 103 | |
|---|---|---|---|---|---|---|
| COMP | M/T | % | M/T | % | M/T | % |
| $H_2$ | 184.93 | 69.31 | 514.11 | 69.60 | 699.04 | 69.51 |
| CO | 43.54 | 16.32 | 76.11 | 10.30 | 119.65 | 11.90 |
| $CO_2$ | 31.95 | 11.97 | 52.09 | 7.05 | 84.04 | 8.36 |
| I | 6.41 | 2.40 | 93.39 | 12.64 | 99.80 | 9.93 |
| MEOH | | | 3.10 | 0.41 | 3.00 | 0.30 |
| TOTAL | 266.83 | 100.00 | 738.80 | 100.00 | 1005.53 | 100.00 |

| STR | 109 | | 112 | 125 |
|---|---|---|---|---|
| COMP | M/T | % | M/T | M/T |
| $H_2$ | 549.31 | 62.40 | 2.13 | 33.27 |
| CO | 81.35 | 9.24 | 0.46 | 4.78 |
| $CO_2$ | 59.73 | 6.79 | 4.34 | 3.30 |
| I | 99.80 | 11.33 | 0.57 | 5.84 |
| MEOH | 65.81 | 7.48 | 62.50 | 0.21 |
| $H_2O$ | 24.31 | 2.76 | 24.31 | |
| TOTAL | 880.31 | 100.00 | 94.31 | 47.40 |

in Example 9) which functions at a pressure of 105 kg/sq.cm. to 527 kg/sq.cm. (specifically at 280—292 for this Example) and at temperatures within the range from about 260°C. to 335°C. to produce methanol from hydrogen and carbon oxide. $C_3$ is a high temperature methanol synthesis catalyst (described in Example 8) which functions at a pressure of 105 kg/sq.cm. to 527 kg/sq.cm. (specifically 280—295 for this example), and at temperatures within the range from about 315°C. to 400°C. to produce methanol from hydrogen and a carbon oxide.

The effluent from reactor 115 flows through line 106 and through indirect heat-exchanger 116 wherein the effluent is cooled by means of indirect heat-exchange with boiler feed water introduced through line 122. After having passed through heat-exchanger 116, the effluent is at a temperature of 246°C. and then mixes with Stream 127.

The mix is introduced into bed 117 containing $C_1$ catalyst and then a bed 118 of $C_2$ catalyst identified in FIG. 1 as assemblage $A_2$.

The effluent from bed 118 passes through line 109 and is at a temperature of 326°C. The stream in line 109 contains 7.48 per cent methanol on a molar basis. At equilibrium, the stream would have contained 21.69 mole per cent methanol. The reaction to produce methanol approaches equilibrium to the extent of 43 per cent, based upon the conversion of carbon monoxide, or 35 per cent of methanol equilibrium.

Stream 109 is cooled by passage through indirect heat-exchanger 126; then through indirect heat-exchanger 119 generating low pressure steam from water introduced by means of line 123; and by passage through air-cooler 120. The process stream exiting cooler 120 is introduced by means of line 110 into separator 121, from the bottom of which liquid methanol product, containing small quantities of dimethyl ether and higher alcohols, admixed with water, is removed through line 112.

A gaseous phase is removed from separator 121 by means of line 124. Part of the stream passing through line 124 is returned into the synthesis loop by means of line 102, and the remainder is purged from the system by means of line 125.

Using the process described in this Example a 4535 metric ton per day methanol plant, would require a total of about 135 cubic meters of catalyst distributed approximately as follows:

Bed 113, Catalyst $C_1$, volume approximately 13.5$m^3$
Bed 114, Catalyst $C_2$, volume approximately 18.9$m^3$
Bed 115, Catalyst $C_3$, volume approximately 40.5$m^3$
Bed 117, Catalyst $C_1$, volume approximately 28.4$m^3$
Bed 118, Catalyst $C_2$, volume approximately 33.7$m^3$

These are approximate volumes of catalyst and pertain to catalysts of the present art. However, as improvements in catalysts are made the quantities of catalyst in the reactor beds or trays, and their distribution can be modified. The benefits to be attained by a more active catalyst are reduced pressure drop and reduced catalyst cost. The catalyst can be six mm. by six mm. by right cylinders. Other sizes and shapes may be preferred under specified circumstances. As a general rule the catalyst volume increases in successive downstream beds in each assemblage because the methanol partial pressure increased and the catalysts are designed for essentially equivalent productivity at equal pressures but at incrementally higher temperatures in the order $C_1$, $C_2$ and $C_3$. Because of the increasing partial pressure of methanol, the catalyst volume must be increased to compensate for the decreased "driving force" for the reaction.

Example 2

In this embodiment of the present invention the synthesis gas is prepared by the gasification of coal and is to be considered in conjunction with FIG. 2 of the drawings and Table II.

Referring to FIG. 2, fresh feed at a pressure of 267 kg/sq.cm. is introduced into the synthesis loop through line 301 and is admixed with recycle gas flowing through line 302. The mixed stream is then pumped by circulating compressor 311 through heat-exchanger 326 wherein the temperature of the gas is raised to 232°C. To produce one short ton of methanol, 50 brake horsepower-hours is supplied to compressor 311.

Stream 303 passes through assemblage $A_1$ consisting of three beds of catalyst $C_1$, $C_2$ and $C_3$ which are located in beds 313, 314 and 315. The temperature of the gas exiting bed 313 is 277°C., the temperature of the gas exiting bed 314 is 335°C., and the temperature of the gas exiting bed 315 is 382°C. The $C_1$, $C_2$ and $C_3$ catalysts are the same as those described in Example 1.

The stream in line 306 contains 4.59 per cent methanol on a molar basis. At equilibrium, the stream would have contained 13.7 mole per cent methanol. The reaction to produce methanol approaches equilibrium to the extent of 39 per cent, based upon the conversion of carbon monoxide, or 35 per cent of methanol equilibrium.

TABLE II

| STR | 301 | | 302 | | 303 | |
|---|---|---|---|---|---|---|
| COMP | M/T | % | M/T | % | M/T | % |
| $H_2$ | 137.53 | 66.85 | 573.27 | 72.18 | 710.80 | 71.08 |
| CO | 65.24 | 31.71 | 114.16 | 14.37 | 179.40 | 17.94 |
| $CO_2$ | 0.66 | 0.32 | 6.34 | 0.80 | 7.00 | 0.70 |
| I | 2.31 | 1.12 | 97.39 | 12.26 | 99.70 | 9.97 |
| MEOH | | | 3.07 | 0.39 | 3.07 | 0.31 |
| TOTAL | 205.74 | 100.00 | 794.23 | 100.00 | 999.97 | 100.00 |

| STR | 305 | | 306 | |
|---|---|---|---|---|
| COMP | M/T | % | M/T | % |
| $H_2$ | 658.36 | 69.48 | 632.36 | 68.62 |
| CO | 153.18 | 16.17 | 140.18 | 15.21 |
| $CO_2$ | 7.00 | 0.74 | 7.00 | 0.76 |
| I | 99.70 | 10.52 | 99.70 | 10.82 |
| MEOH | 29.29 | 3.09 | 42.29 | 4.59 |
| TOTAL | 947.53 | 100.00 | 921.53 | 100.00 |

| STR | 309 | | 312 | 325 |
|---|---|---|---|---|
| COMP | M/T | % | M/T | M/T |
| $H_2$ | 585.70 | 66.94 | 2.37 | 10.06 |
| CO | 116.85 | 13.36 | 0.69 | 2.00 |
| $CO_2$ | 7.00 | 0.80 | 0.56 | 0.10 |
| I | 99.70 | 11.40 | 0.60 | 1.71 |
| MEOH | 65.62 | 7.50 | 62.50 | 0.05 |
| TOTAL | 874.87 | 100.00 | 66.72 | 13.92 |

The effluent from bed 315 flows through line 306 and through indirect heat-exchanger 316 wherien the effluent is cooled by means of indirect heat-exchange with boiler feed water introduced through line 322. After having passed through heat-exchanger 316, the effluent is at a temperature of 232°C.

The effluent is then introduced into assemblage $A_2$ consisting of bed 317, initially contacting a bed of $C_1$ catalyst and then a bed, 318, of $C_2$ catalyst, those catalysts having been defined in Example 1.

The effluent from bed 318 passes through line 309 and is at a temperature of 315°C. The stream in line 309 contains 7.50 per cent methanol on a molar basis. At equilibrium, the stream would have contained 25.5 mole per cent methanol. The reaction to produce methanol approaches equilibrium to

the extent of 39 per cent, based upon the conversion of carbon monoxide, or 29 per cent of methanol eqiulibrium.

Stream 309 is cooled by passage through indirect heat-exchanger 326, by passage through indirect heat-exchanger 319, generating low pressure stream from boiler feed water introduced by means of line 323, and by passage through air cooler 320. The process stream exiting cooler 320 is introduced into separator 321, from the bottom of which liquid methanol, containing small quantities of dimethyl ether and higher alcohols and which is almost anhydrous, is removed through line 312.

A gaseous phase is removed overhead from separator 321 through line 324 and split into streams 302 and 325. Stream 302 is returned into the synthesis loop, whereas stream 325 is purged.

Using the process of this example a 4535 metric ton per day methanol plant would require about 135 cubic meters of catalyst distributed approximately as set forth in Example 1.

Example 3

This Example describes an embodiment of the present invention in which the synthesis gas is prepared by the gasification of coal and is to be considered in conjunction with FIG. 3 of the drawings and Table III.

Referring to FIG. 3, fresh feed at a pressure of 295 kg/sq.cm. is introduced into the synthesis loop through line 501 and is admixed with recycle gas flowing through line 502. The mixed stream is compressed to a pressure of 316 kg/sq.cm. by means of compressor 512. The compressed stream flows through heat-exchanger 523 wherein the temperature of the gas is raised to 200°C. To produce one short ton of methanol, 27 brake horse power hours is supplied to compressor 512.

Stream 503 passes through three beds of catalyst $C_1$, $C_2$ and $C_3$, which are contained in beds 513, 514 and 515, and are identified as assemblage $A_1$.

The temperature of the gas leaving bed 513 is 260°C., the temperature of the gas leaving bed 514 is 330°C. and the temperature of the gas leaving bed 515 is 388°C. The $C_1$, $C_2$ and $C_3$ catalysts are the same as those described in Example 1.

The stream in line 506 contains 6.0 per cent methanol on a molar basis. At equilibrium the stream would have contained 13.6 mole per cent methanol. The reaction to produce methanol approaches equilibrium to the extent of 47 per cent, based upon the conversion of carbon monoxide, or 44 per cent of methanol equilibrium.

TABLE III

| STR | 501 | | 502 | | 503 | |
|---|---|---|---|---|---|---|
| COMP | M/T | % | M/T | % | M/T | % |
| $H_2$ | 136.63 | 67.07 | 287.90 | 73.49 | 424.53 | 71.27 |
| CO | 64.40 | 31.50 | 42.07 | 10.76 | 106.47 | 17.88 |
| $CO_2$ | 0.62 | 0.30 | 2.98 | 0.76 | 3.60 | 0.61 |
| I | 2.31 | 1.13 | 57.09 | 14.60 | 59.40 | 9.98 |
| MEOH | | | 1.54 | 0.39 | 1.54 | 0.26 |
| TOTAL | 203.96 | 100.00 | 391.58 | 100.00 | 595.54 | 100.00 |

| STR | 504 | 505 | 506 | | 507 | 508 |
|---|---|---|---|---|---|---|
| COMP | M/T | M/T | M/T | % | M/T | M/T |
| $H_2$ | 406.03 | 382.85 | 363.45 | 68.03 | 344.93 | 321.75 |
| CO | 97.22 | 85.63 | 75.93 | 14.20 | 66.67 | 55.08 |
| $CO_2$ | 3.60 | 3.60 | 3.60 | 0.67 | 3.60 | 3.60 |
| I | 59.40 | 59.40 | 59.40 | 11.11 | 59.40 | 59.40 |
| MEOH | 10.79 | 22.38 | 32.08 | 5.99 | 41.34 | 52.93 |
| TOTAL | 577.04 | 553.86 | 534.46 | 100.00 | 515.94 | 492.76 |

| STR | 509 | | 510 | 511 |
|---|---|---|---|---|
| COMP | M/T | % | M/T | M/T |
| $H_2$ | 299.43 | 63.66 | 9.12 | 2.41 |
| CO | 43.92 | 9.34 | 1.33 | 0.52 |
| $CO_2$ | 3.60 | 0.77 | 0.09 | 0.53 |
| I | 59.40 | 12.63 | 1.81 | 0.50 |
| MEOH | 64.09 | 13.60 | 0.05 | 62.50 |
| TOTAL | 470.44 | 100.00 | 12.40 | 66.46 |

The effluent from bed 515 flows through line 506 and through indirect heat-exchanger 516 wherein the effluent is cooled by means of indirect heat-exchange with boiler feed water flowing through line 517. After having passed through heat-exchanger 516, the effluent is at a temperature of 200°C.

The effluent is then introduced into assemblage $A_2$ consisting of bed 518, initially contacting a bed of $C_1$ catalyst and then bed 519 of $C_2$ catalyst, those catalysts having been defined in Example 1.

The effluent from bed 519 is at a temperature of 330°C. The effluent from bed 519 flows through line 508 and through indirect heat-exchanger 520 wherein the effluent is cooled by means of indirect heat exchange with boiler feed water, introduced through line 521. After having passed through heat-exchanger 520, the effluent is at a temperature of 200°C.

The effluent is then introduced into assemblage $A_3$, which is bed 522, there contacting the $C_1$ catalyst, as described in Example 1.

The effluent from bed 522 passes through line 509 and is at a temperature of 270°C. The stream in line 509 contains 13.6 per cent methanol on a molar basis. At equilibrium, the stream would have contained 27.5 mole per cent methanol. The reaction to produce methanol approaches equilibrium to the extent of 60 per cent, based upon the conversion of carbon monoxide, or 50 per cent of methanol equilibrium.

Stream 509 is cooled by passage through indirect heat-exchanger 523, by passage through indirect heat-exchanger 524 generating low pressure steam from boiler feed water introduced by means of line 527, and by passage through air cooler 525. The process stream leaving cooler 525 is introduced into separator 526 from the bottom of which liquid methanol, containing small quantities of dimethyl ether and higher alcohols, which is almost anhydrous, is removed through line 511.

A gaseous phase is removed overhead from separator 526 through line 528 and splits into streams 502 and 510. Stream 502 is returned into the synthesis loop, whereas stream 510 is purged.

Employing this Example, a 4535 metric ton per day methanol plant will require about 150 cubic meters of catalyst. Because these catalysts will be design by functioning at different temperatures and with different methanol partial pressures, as explained in Examples 1 and 2, the types and quantities will be distributed approximately as follows:

Bed 513, Catalyst $C_1$, volume approximately 10.9$m^3$
Bed 514, Catalyst $C_2$, volume approximately 16.6$m^3$
Bed 515, Catalyst $C_3$, volume approximately 27.1$m^3$
Bed 518, Catalyst C1, volume approximately 24.9$m^3$
Bed 519, Catalyst $C_2$, volume approximately 29.9$m^3$
Bed 522, Catalyst $C_1$, volume approximately 40.6$m^3$

Example 4

This Example describes an embodiment of this invention in which the synthesis gas is derived from coal and is to be considered in conjunction with FIG. 4 and Table IV.

Referring to FIG. 4, fresh feed (make-up gas) is introduced into the synthesis loop at a pressure 93 kg/sq.cm. through line 601 and is admixed with recycle gas flowing through line 602.

TABLE IV

| STR | 601 | | 602 | |
|---|---|---|---|---|
| COMP | M/T | % | M/T | % |
| $H_2$ | 139.61 | 67.08 | 930.22 | 71.51 |
| CO | 65.87 | 31.65 | 204.38 | 15.71 |
| $CO_2$ | 0.32 | 0.15 | 10.24 | 0.79 |
| I | 2.33 | 1.12 | 148.11 | 11.39 |
| MEOH | | | 7.85 | 0.60 |
| TOTAL | 208.13 | 100.00 | 1300.80 | 100.00 |

| STR | 603 | | 604 |
|---|---|---|---|
| COMP | M/T | % | M/T |
| $H_2$ | 1069.83 | 70.90 | 1026.27 |
| CO | 270.25 | 17.91 | 248.47 |
| $CO_2$ | 10.56 | 0.70 | 10.56 |
| I | 150.44 | 9.97 | 150.44 |
| MEOH | 7.85 | 0.52 | 29.63 |
| TOTAL | 1508.93 | 100.00 | 1465.37 |

| STR | 605 | | 606 | |
|---|---|---|---|---|
| COMP | M/T | % | M/T | % |
| $H_2$ | 986.95 | 69.21 | 944.59 | 68.27 |
| CO | 228.81 | 16.04 | 207.63 | 15.01 |
| $CO_2$ | 10.56 | 0.74 | 10.56 | 0.76 |
| I | 150.44 | 10.55 | 150.44 | 10.87 |
| MEOH | 49.29 | 3.46 | 70.47 | 5.09 |
| TOTAL | 1426.05 | 100.00 | 1383.69 | 100.00 |

| STR | 607 | 608 |
|---|---|---|
| COMP | M/T | M/T |
| $H_2$ | 0.77 | 13.60 |
| CO | 0.26 | 2.99 |
| $CO_2$ | 0.17 | 0.15 |
| I | 0.17 | 2.16 |
| MEOH | 62.50 | 0.12 |
| TOTAL | 63.87 | 19.02 |

The mixed gas stream is then pumped by circulating compressor 609 through heat-exchanger 615, wherein the temperature of the gas is raised to 232°C. To produce 0.91 metric ton of methanol, 101 brake horse power hours is supplied to compressor 609. Stream 603 passes through heat-exchanger 615, where it is heated to 232°C. as previously stated, and then for best results, can pass through a vessel (not shown in FIG. 4) containing a catalyst poison removal adsorbent.

Stream 603, after purification, then passes through assemblage $A_1$ of two beds of catalyst, C—1 and C—2, which are contained in beds 610 and 611. The temperature of the gas leaving bed 610 is 280°C. and the temperature of the gas leaving bed 611 is 328°C., the temperature increasing because

of the exothermal methanol reaction occurring in each catalyst bed. C—1 is a low temperature methanol synthesis catalyst which functions efficiently at pressures of 70 kg/sq.cm. to 527 kg/sq.cm. and at temperatures in the range of 200°C. This C—1 catalyst, as well as the C—2 and C—3 catalysts, have previously been described. After passing through beds 610 and 611, the temperature of the effluent of bed 611 is 328°C. The effluent contains 3.46 mol % methanol, which is a 49.4% approach to equilibrium, based on CO conversion.

The effluent from bed 611 flows through line 605 and then through indirect heat-exchanger 612, wherein the effluent is cooled by means of the indirect heat-exchanger with boiler feed water introduced through line 613. After having passed through the heat-exchanger 612, the effluent gas is at a temperature of 232°C.

The gas is next introduced into assemblage $A_2$, which is one bed 614 containing C—1 catalyst, from which it emerges at 280°C. The stream now in line 606 contains 5.09% methanol on a molar basis. At equilibrium the stream would contain 17.72% methanol. The reaction to produce methanol approaches equilibrium to the extent of 35.50%, based on the conversion of carbon monoxide.

Stream 606 is cooled by passage through indirect heat-exchanger 615, which preheats the gas for inlet to bed 610, and the gas in line 606 is thereby cooled to 109°C. The gas then passes through boiler feed water preheater 616, preheating the boiler feed water entering through line 617. The effluent gas is at 93°C. and passes into air cooler 618, which condenses the methanol and other products such as higher alcohols and dimethyl ether (in solution in the methanol). This gas-liquid mixture in line 619 passes into the separator 621. The liquid is removed from the separator through line 607, whereas the gas leaves via line 620. The gas leaving via 620 is divided into two streams, one passing through line 608 to purge and the balance passing through line 602 to the inlet to the recirculation pump to be recycled to the beds.

Following this example, a 4539 metric ton per day methanol plant would require about 205 cubic meters of catalyst. Because of the lower pressure of this example, the quantity of catalyst should be approximately 50% greater. This larger volume of catalyst will be distributed approximately as follows:

Bed 610, Catalyst $C_1$, volume approximately 55m$^3$
Bed 611, Catalyst $C_2$, volume approximately 68m$^3$
Bed 614, Catalyst $C_1$, volume approximately 82m$^3$

Example 5

This Example describes an embodiment of this invention in which the synthesis gas is derived from coal and is to be considered in conjunction with FIG. 5 and Table V.

Referring to FIG. 5, fresh feed (make-up gas) is introduced into the synthesis loop at a pressure of 435 kg/sq.cm. through line 701 and is admixed with recycle gas flowing through line 702. The mixed gas stream is then pumped by circulating compressor 713 through heat-exchanger 725, wherein the temperature of the gas is raised to 232°C. To produce 0.91 metric tons of methanol, 32 brake horse power hours is supplied to the compressor 713. Stream 703 passes through heat-exchanger 725 where, as previously stated, it is heated to 232°C., and then for best results can pass through a vessel (not shown in FIG. 5) containing a poison removal adsorbent.

Stream 703, after purification, passes through three beds of catalyst, C—1, C—2 and C—3, which are located in beds 714, 715 and 716, respectively, which comprise assemblage $A_1$. The temperature of the gas leaving bed 714 is 280°C., that leaving bed 715 is 335°C. and that leaving bed 716 is 388°C. The temperature increases because of the exothermal methanol synthesis reaction occuring in each catalyst bed. C—1 is the low temperature methanol catalyst which functions efficiently and without wax formation at pressures of 70 to 527 kg/sq.cm. and at temperatures in the range of 200°C. to 285°C. This C—1 catalyst, as well as the C—2 and C—3 catalysts, have been previously described herein, but will be described more fully in Examples 8, 9 and 10. After passing through the reactors 714, 715 and 716, the temperature of the effluent from reactor 716 is, as previously noted, 388°C. The gas contains 4.57 mol % methanol and is at 29.7% of approach to equilibrium, based on the CO conversion.

TABLE V

| | 701 | | 702 | | 703 | |
|---|---|---|---|---|---|---|
| COMP | M/T | % | M/T | % | M/T | % |
| $H_2$ | 135.75 | 66.76 | 367.27 | 73.11 | 503.02 | 71.28 |
| CO | 64.63 | 31.78 | 62.40 | 12.42 | 127.03 | 18.00 |
| $CO_2$ | 0.70 | 0.34 | 3.25 | 0.65 | 3.95 | 0.56 |
| I | 2.28 | 1.12 | 68.08 | 13.55 | 70.36 | 9.97 |
| MEOH | | | 1.34 | 0.27 | 1.34 | 0.19 |
| TOTAL | 203.36 | 100.00 | 502.34 | 100.00 | 705.70 | 100.00 |

| STR | 704 | 705 | 706 | | 707 | 708 |
|---|---|---|---|---|---|---|
| COMP | M/T | M/T | M/T | % | M/T | M/T |
| $H_2$ | 485.40 | 467.14 | 446.40 | 68.77 | 428.98 | 408.96 |
| CO | 118.22 | 109.09 | 98.72 | 15.21 | 90.01 | 80.00 |
| $CO_2$ | 3.95 | 3.95 | 3.95 | 0.61 | 3.95 | 3.95 |
| I | 70.36 | 70.36 | 70.36 | 10.84 | 70.36 | 70.36 |
| MEOH | 10.15 | 19.28 | 29.65 | 4.57 | 38.36 | 48.37 |
| TOTAL | 688.08 | 669.82 | 649.08 | 100.00 | 631.66 | 611.64 |

| STR | 709 | 710 | | 711 | 712 |
|---|---|---|---|---|---|
| COMP | M/T | M/T | % | M/T | M/T |
| $H_2$ | 392.34 | 377.96 | 65.09 | 6.95 | 3.74 |
| CO | 71.69 | 64.50 | 11.11 | 1.18 | 0.92 |
| $CO_2$ | 3.95 | 3.95 | 0.68 | 0.06 | 0.64 |
| I | 70.36 | 70.36 | 12.12 | 1.29 | 0.99 |
| MEOH | 56.68 | 63.87 | 11.00 | 0.03 | 62.50 |
| TOTAL | 595.02 | 580.64 | 100.00 | 9.51 | 68.79 |

The effluent from reactor 716 flows through line 706 and through indirect heat-exchanger 717, wherein the effluent is cooled by indirect contact with boiler feed water introduced through line 718. After passing through the heat-exchanger 717, the effluent gas is at a temperature of 280°C.

The gas at 280°C. enters assemblage $A_2$ consisting of bed 719 charged with C—2 catalyst, from which it emerges at 335°C., and enters bed 720 charged with C—3 catalyst, from which it emerges at 388°C. The gas at this point contains 7.91 mol% methanol and is at a 48.5% approach to equilibrium, based on CO Conversion.

The gas exits from bed 720 through line 708 and enters indirect heat-exchanger 721 where boiler

feed water entering through line 722 is preheated, and the gas is thereby cooled to 232°C. as it emerges from the heat-exchanger.

The gas at 232°C. enters assemblage $A_3$ which consists of bed 723 charged with C—1 catalyst, from which it emerges at 280°C. and then enters bed 724 charged with C—2 catalyst, from which it emerges in line 710 and is at 324°C. The gas at this point contains 11.00 mol% methanol and is at 51.4% approach to equilibrium based on CO conversion.

Stream 710 is cooled by passage through indirect heat-exchanger 725, which preheats the inlet gas in line 703, which is the feed to bed 714, the first in the sequence of beds. The cooled gas exiting from heat-exchanger 725 passes into indirect heat-exchanger 726 into which boiler feed water enters through line 729 and is preheated. The gas, on the other hand, is cooled to 93°C. and then for further cooling enters air cooler 727, from which it emerges in line 728 with the methanol at this point being condensed to the liquid phase together with any higher alcohols and other products including dimethyl ether which would be dissolved in the liquid phase.

The gas and liquid are separated in vessel 731 from which the crude methanol is removed through line 712. The gas phase is removed from the separator through line 730. The gas phase is separated into two streams, one passing through line 711 to be purged from the synthesis loop, whereas the other portion passes through line 702 to the compressor 713 for compression and entry into line 703 for recycle to the synthesis converter system.

Following this example, a 4535 metric ton per day methanol plant would require approximately 135 cubic meters of catalysts. At the elevated pressure, there is a likelihood that a lesser quantity could be used, but because seven beds (or trays) are used, it would be essential to have sufficient catalyst on each tray to ensure adequate residence time and avoidance of channeling. These factors would be important considerations in choosing catalyst volume, which could consequently be greater than that strictly required on a theoretical basis.

Bed 714, Catalyst $C_1$, volume approximately 8.1 $m^3$
Bed 715, Catalyst $C_2$, volume approximately 10.8 $m^3$
Bed 716, Catalyst $C_3$, volume approximately 21.6 $m^3$
Bed 719, Catalyst $C_2$, volume approximately 16.1 $m^3$
Bed 720, Catalyst $C_3$, volume approximately $21.6^3$
Bed 723, Catalyst $C_1$, volume approximately 24.3 $m^3$
Bed 724, Catalyst $C_2$, volume approximately 32.4 $m^3$

## Example 6

This Example describes the preparative procedure for one of the catalysts suitable for service at C—1, the low temperature catalyst of the invention. It also describes in less detail or enumerates other catalysts equally satisfactory and which may have special properties for special conditions of presence or absence of $CO_2$ in the gas stream and the desirability of producing higher alcohols or dimethyl ether.

The preparative procedure for one of the most suitable C—1 catalysts is as follows:

a. Dissolve 1440 parts by weight of copper nitrate trihydrate and 297 parts by weight of zinc nitrate hexahydrate and 500 parts by weight of aluminium nitrate nonahydrate in 9,000 parts by weight of distilled water at 60°C with vigorous agitation.

b. While maintaining the temperature at the 60°C level, add ammonium carbonate as a solid over a period of one to two hours to the solution prepared in item 1 above until a pH of 6.6 to 6.8 has been reached.

c. Continue the agitation and maintain the temperature at 60°C for an additional two hour digestion period.

d. After the digestion period filter the slurry and wash the filter cake on the filter with distilled water to remove ammonium nitrate, which would otherwise decompose and cause problems in ensuing operations. The copper, zinc, aluminum and ammonium salts should be free of iron, nickel and other foreign and harmful ions.

e. Dry the filter cake at 150°C.

f. Calcine the dry filter cake for 2 hours at 400°C.

g. Convert the dry filter cake to powder and then prepare pellets, preferably by first densifying, then mixing the densified powder with a pilling lubricant, and then finally pilling to one-fourth inch right cylinders.

h. Finally, heat treat the pills in air at 400°C. for two hours to increase porosity.

i. The pills so produced are then converted to useful methanol synthesis catalyst by reduction to reduced form of copper and zinc, using hydrogen as the reducing agent. The zinc reduces very little, whereas the copper is almost completely reduced. Reduction can be effected within the bed, or preferably externally, and then the catalyst can be introduced to form the bed as a stabilized catalyst. Reduction must be effected carefully with pure hydrogen diluted with an inert gas so as to avoid overheating during reduction. Reduction can be effected at 200°C to 300°C.

Instead of the 6 to one copper to zinc atomic ratio called for in paragraph a. above, ratios of from 8 to one copper atoms per atom of zinc can be used.

Instead of the eleven weight % aluminum oxide stipulated in paragraph a. above, one can use, instead, from two to as much as 99%, and instead of the aluminum being added as nitrate it can also be added as alumina hydrate in finely divided form.

Instead of the ammonium carbonate specified in paragraph b. above, ammonia (anhydrous) with carbon dioxide, or ammonimum bicarbonate can be used. Sodium carbonate also can be used, but if one of the non-volatile carbonates is used, the powder after calcining must be slurried in a solution of ammonium bicarbonate at 0.1 to 1% weight concentration for an ion-exchange of the sodium with ammonium ion. The slurrying must be repeated until the sodium ion has been almost completely removed (less than 50 ppm in the finished catalyst).

Instead of the aluminum oxide, one can substitute other stabilizer-promoters, such as chromium (as trivalent or hexavalent with appropriate changes in preparative procedure), magnesium aluminum (spinel), cerium, rare earth mixtures, lanthanum, praseodymium, neodymium, zirconium, titanium, silicon, niobium and tantalum as the oxides or mixed oxides or solid state reaction products, used in varying ratios in the finished catalyst.

Instead of the nitrate salts of the copper, zinc and stabilizer (alumina), one can use the sulfate, chloride, acetate or other salts and, as in the case of the substitution of the sodium carbonate, it is necessary to slurry the calcined powder in ammonium bicarbonate solution to effect ion-exchange so that the foreign ion such as sulfate or chloride can be below the required 100 parts per million by weight in the finished catalyst. Even with the ion-exchange, the catalyst is noticeably less active than that prepared from the nitrate salts and ammonium carbonate precipitant.

Example 7

This Example describes the preparative procedure for the catalyst designated as C—2 and is the one for the intermediate temperature range for methanol synthesis.

The procedure used in Example 6 is employed except that only 960 parts by weight of copper nitrate trihydrate and 1188 parts of zinc nitrate hexahydrate are used. The aluminum nitrate nonahydrate parts by weight remain the same. This produces a one to one copper to zinc atomic ratio in the final catalyst, except for copper lost to amine during the precipitation and subsequent filtration and washing.

The copper to zinc atomic ratio should be maintained in the range of from 0.5 to preferably 4 atoms of zinc per atom of copper.

Example 8

This Example describes the catalyst suitable for use in the high temperature bed, the catalyst designated C—3. This catalyst must be capable of withstanding high temperatures and must also be capable of effecting the reverse shift: $CO_2 + H_2 = CO + H_2O$. Consequently, there are conditions under which a mixed catalyst charge is preferred, and these will be described in this example. At least 5% of a catalyst especially effective for reverse shift reaction should be present in a mixed catalyst charge.

The single preferred catalyst, however, is made as follows:

a. Dissolve 482 parts by weight of copper nitrate trihydrate, 1188 parts by weight of zinc nitrate hexahydrate and 630 parts by weight of chromic acid anhydride ($CrO_3$) in 9000 parts by weight of distilled water. As above, all salt should be as free from iron and nickel contamination as possible. Adjust the temperature to 60°C. and agitate vigorously.

b. While maintaining the temperature at 60° to 70°C. and while agitating vigorously, add anhydrous ammonia to the solution of item 1 over a period of about one hour until a pH of 6.6 to 6.8 has been reached.

c. Continue agitation, but let the temperature drift lower for an additional two hours after the precipitation has been completed.

d. After the "digestion" period of item 3, the slurry is filtered and the filter cake is washed on the filter with distilled water to remove the ammonium nitrate which would otherwise decompose and cause problems in ensuing operations.

e. Dry the filter cake at 150°C.

f. Calcine the dry cake at 450°C for 2 hours and then powder the dry cake.

g. Convert the powder of item 1 to pellets, preferably by first densifying, mixing with a pilling lubricant, and then pilling to right cylinders 1/4 inch by 1/4 inch.

h. Finally, heat treat the pills in air 500°C for two hours to increase porosity.

i. The pills so produced can now be converted to a useful methanol synthesis catalyst with excellent reverse shift characteristics by reduction using dilute hydrogen to produce almost elemental copper and partially reduced zinc. The reduction must be conducted very carefully because of the presence of some chromium in hexavalent oxide form. Reduction can be effected at 250°C to 350°C. Reduction can be effected in the bed, or preferably externally, and then stabilizing for transer to the converter.

A satisfactory catalyst can be produced also using chromic acid and dissolving a stoichiometric or greater quantity of zinc therein. It is an inexpensive catalyst, but it is not effective for the reverse carbon monoxide shift. When this catalyst is used, it is preferable to mix equal portions of the first catalyst

described in this example with it. It is also possible to mix some of the catalysts described for C—2 in it on a one to one volume basis.

As stated in Example 6, salts other than nitrates can be used, but any offending ions must be eliminated in the finished catalyst by washing with ammonium bicarbonate solution to remove the alkali ions as well as the anions such as C1 and $CO_4$.

These catalysts in this Example 8 can also be stabilized by the co-precipitation of the stabilizers given in Example 6. These, under the higher temperature conditions pertaining for the environment for this catalyst C—3, can also act in some case as promoters. Ceria and zirconia can, for example, beneficially affect the C—3 catalyst in its service. The weight ratio of stabilizer to catalyst can be in the range of 1 to 80 parts of stabilizer to 100 parts of catalyst.

Instead of the copper to zinc ratio of 1 to 2 stipulated in the step a of this Example 7, the atom ratio can be 1 to 1 or as high as 1 to 6 (i.e., one Cu to six Zn) with appropriate changes in the chromic acid anhydride requirement.

Instead of the chromic acid anhydride, one can use a stoichiometric quantity of chromium nitrate. Furthermore, one can substitute the stabilizer promoters of Example 6 for the chromium (e.g., ceria, thoria, lanthana, zirconia, titania, mixed rare earth oxides, magnesia, silica, tantala, alumina, niobia, finely divided spinels, etc.). These can of course be added in varying ratios and can be intermixed and interreacted.

A catalyst C—3 for the third bed, capable of producing higher alcohols includes a portion of manganese. The quantity of manganese can affect the quantities of higher alcohols produced, with the higher proportions of manganese producing higher quantities of higher alcohols. The manganese, copper, zinc and promoters and stabilizers are co-precipitated from the nitrates as described for example 8, the C—1 catalyst. Copper can be eliminated and so can the zinc, but a preferred atomic ratio is 1 Cu: 2 Zn: 0.5 Al, all as oxides. Instead of the Al, Ce, Zr and mixed rare earths, all as oxides, can be used. The catalyst is pelleted and heat treated as described in steps g. and h.

Alkalis (sodium, potassium, rubidium and cesium) as carbonate, permanganate, chromate or silicate, for example, can also be added to a catalyst as promoters intended for enhanced higher alcohol production. The silica as silicate or as colloidal silica is also useful if it is desired to make increased quantities of dimethyl ether or silimar oxygenated products. These can be added during the aforementioned densification operation.

It will be apparent to one skilled in the art that instead of the catalysts being housed in separate converters, as described in the foregoing examples, these catalysts can be all housed in a single converter, with the catalysts contained in beds separated by solid plates in the converter. These plates would force the gas into exit lines passing into heat-exchangers and from the heat-exchangers back into a down-stream bed where reaction again takes place. The gas effluent from any given bed can again be forced from the converter, via a solid plate, into external heat-exchanger (recover) and back to one or more additional beds or, if desired, passed out of the converter for heat recovery and methanol condensation.

## Claims

1. In the process for the synthesis of methanol wherein an original gaseous mixture containing hydrogen and at least one of the oxides of carbon is passed under conditions of elevated temperature and pressure into contact with a plurality of catalysts in succession, the improvement which comprises the steps of:

(a) passing said original gaseous mixture through a first assemblage of two or more beds of different catalysts arranged in series, the catalyst in each of said beds being of such composition as to function most effectively to produce methanol in a lower temperature range than will the catalyst in each succeeding bed, each of which is in a higher temperature range, said original mixture being introduced into the first bed at a temperature within the range from 200°C. to 260°C. and the gaseous mixture resulting, after passing through said beds, exits the last bed at a temperature within the range from 370°C. to 400°C.,

(b) cooling the gaseous stream exiting from step (a) to a temperature within the range from 200°C. to 260°C. by indirect heat-exchange, and

(c) passing the gaseous stream exiting from step (b) through a second assemblage of one or more beds each charged with a particular catalyst selected to function most effectively to produce methanol within the particular temperature range of the gaseous stream prevailing therein, whereby the temperature of the gas stream exiting from step (b) is raised to from 285°C. to 345°C. by the heat of the methanol producing reaction.

2. The process of claim 1 which is carried out at an operating pressure within the range from 70 kg/sq.cm. to 527 kg/sq.cm.

3. The process of claim 1 wherein the original gaseous mixture contains carbon monoxide and carbon dioxide and is composed of at least two-thirds carbon monoxide by volume, based upon the total volume of carbon monoxide and carbon dioxide.

4. The process of claim 1 for synthesis of methanol from hydrogen and carbon monoxide wherein in step (a) said first assemblage of catalyst beds consists of three individual beds of catalysts arranged

in series, the bed initially contacted by the original gaseous mixture being composed of catalyst which functions most effectively over temperatures within the range from 200°C to 285°C., the second individual bed contacted by the stream of gases being composed of catalyst which functions most effectively over temperatures within the range from 260°C. to 335°C. the third individual bed contacted by the stream of gases being composed of catalyst which functions over temperatures within the range from 315°C. to 400°C. and wherein the step (c) said second assemblage consists of two beds of catalysts arranged in series, the individual bed first contacted by the stream of gases in step (c) being composed of catalyst which functions most effectively over temperatures within the range from 200°C. to 285°C. and the second individual bed contacted by the stream of gases being composed of catalyst which functions most effectively over temperatures within the range from 260°C. to 345°C.

5. In the process for the synthesis of methanol wherein an original gaseous mixture containing hydrogen and at least one of the oxides of carbon is passed into contact with a plurality of catalysts in succession under conditions of elevated temperature and pressure the improvement which comprises the steps of:

(a) passing said original gaseous mixture through a first assemblage of three beds of different catalysts arranged in series, the catalyst in each of said beds being of such composition as to function most effectively to produce methanol in a lower temperature range than will the catalyst in each succeeding bed, each of which is in a higher temperature range, said original mixture being introduced into the first bed at a temperature within the range from 200°C. to 260°C. and the gaseous mixture resulting, after passing through said beds, exits the third bed at a temperature within the range from 370°C. to 400°C.,

(b) cooling the gaseous stream exiting from step (a) to a temperature within the range from 200°C. to 260°C. by indirect heat-exchange,

(c) passing the gaseous stream exiting step (b) through a second assemblage of two individual beds of different catalysts arranged in series, the catalyst in the first of such individual beds being of such composition as to function most effectively at a lower temperature range than the catalyst in the second of such beds, the said gaseous stream existing from step (b) being introduced into the first of the beds of said second assemblage at a temperature within the range from 200°C. to 260°C. and exiting the second of the beds of said second assemblage at a temperature within the range from 260°C. to 345°C.,

(d) cooling the gaseous stream existing from step (c) to a temperature within the range from 200°C. to 260°C. by indirect heat-exchange, and,

(e) passing the gaseous stream exiting from step (d) through one final bed of catalyst, said gaseous stream exiting from step (d) being introduced into said final bed at a temperature within the range from 200°C. to 260°C. and exiting said final bed at a temperature within the range from 260°C. to 285°C.

6. The process of claim 5 to produce methanol from hydrogen and carbon monoxide wherein in steps (a) and (c) the bed of catalyst initially contacted by the stream in each of the first assemblage and the second assemblage of beds is composed of catalysts which function most effectively over temperatures within the range from 200°C. to 285°C. wherein the steps (a) and (c) the next beds of catalysts in the first and second assemblage of beds are composed of a catalyst which functions most effectively over temperatures within the range from 260°C. to 335°C.; and wherein in step (a) the third bed of catalyst in the first assemblage of beds which in contacted by the respective gaseous stream is composed of a catalyst which functions most effectively over temperatures within the range from 315°C. to 400°C.

7. The process of claim 1 wherein the majority of said individual beds of catalysts contain compounds of copper and zinc.

8. The process of claim 4 wherein in steps (a) and (c) the first bed of each of the first and the second assemblages of beds which is contacted by the gaseous mixture contains a catalyst which is comprised of from one to six atoms of copper per atom of zinc.

9. The process of claim 5 wherein in steps (a), (c) and (e) the bed initially contacted by the gaseous mixture contains catalyst which is comprised of from one to six atoms of copper per atom of zinc.

10. The process of claim 4 wherein in steps (a) and (c) the second bed contacted by the gaseous mixture contains catalyst which is comprised of from 0.5 to 4 atoms of zinc per atoms of copper.

11. The process of claims 5 wherein in step (a) the third bed contacted by the gaseous mixture contains catalyst which is comprised of from one to six atoms of zinc per atom of copper.

12. The process of claim 1 wherein the catalyst is comprised of a stabilizing amount of one or more oxides selected from the group consisting of aluminum, chromium, magnesium, cerium, rare earth mixtures, lanthanum, praseodymium, neodymium, zirconium, titanium, silicon, niobium or tantalum or a mixture thereof.

13. The process of claim 5 wherein in step (e) the catalyst is comprised of manganese as oxide in amounts sufficient to promote the production of higher alcohols.

14. The process of claim 4, wherein steps (a) and (c), the first two beds contacted by the gaseous mixture contain catalysts which are comprised of copper and zinc; and wherein step (a) the third bed contacted by the gaseous mixture contains catalyst which is comprised of zinc.

**Revendications**

1. Procédé pour la synthèse de méthanol, dans lequel on fait passer un mélange gazeux de départ, contenant de l'hydrogène et au moins l'un des oxydes de carbone, dans des conditions de température et de pression élevées, au contact de plusieurs catalyseurs successivement, caractérisé par les opérations successives consistant à:

(a) faire passer le mélange gazeux de départ à travers un premier ensemble de deux ou de plusieurs lits de catalyseurs différents disposés en série, le catalyseur de chacun de ces lits ayant une composition telle qu'il ait son maximum d'activité pour la production de méthanol dans une gamme de température plus basse que le catalyseur du lit immédiatement suivant, qui est à une température plus élevée, le mélange de départ étant introduit dans le premier lit à une température se situant dans la gamme comprise entre 200°C et 260°C, et le mélange gazeux résultant sortant du dernier lit, après être passé à travers les différents lits, à une température se situant dans la gamme comprise entre 370°C et 400°C,

(b) refroidir le courant gazeux qui sort de l'étage (a) à une température se situant dans la gamme comprise entre 200°C et 260°C par échange de chaleur indirect, et

(c) faire passer le courant gazeux qui sort de l'étage (b) à travers un second ensemble d'un ou de plusieurs lits, chargés chacun d'un catalyseur particulier, choisi de telle manière que son maximum d'activité pour la production de méthanol se situe dans la gamme de température particulière du courant gazeux qui y règne, ce qui fait que la température du courant de gaz qui sort de l'étage (b) est élevée de 285°C à 345°C par la chaleur de la réaction de production du méthanol.

2. Procédé selon la revendication 1, caractérisé en ce qu'il est exécuté à une pression opératoire se situant dans la gamme comprise entre 70 kg/cm$^2$ et 527 kg/cm$^2$.

3. Procédé selon la revendication 1, caractérisé en ce que le mélange gazeux de départ contient de l'oxyde de carbone et de l'anhydride carbonique et est composé d'au moins deux tiers d'oxyde de carbone en volume, sur la base du volume total d'oxyde de carbone et d'anhydride carbonique.

4. Procédé selon la revendication 1 pour la synthèse de méthanol à partir d'hydrogène et d'oxyde de carbone, caractérisé en ce que dans l'étage (a), le premier ensemble de lits de catalyseurs se compose de trois lits séparés de catalyseurs disposés en série, le lit avec lequel entre tout d'abord en contact le mélange gazeux de départ étant fait d'un catalyseur dont le maximum d'activité se situe à des températures comprises dans la gamme de 200°C à 285°C, le deuxième lit avec lequel entre en contact le courant de gaz étant fait d'un catalyseur dont le maximum d'activité se situe à des températures comprises dans la gamme de 260°C à 335°C, le troisième lit avec lequel entre en contact le courant de gaz étant fait d'un catalyseur dont le maximum d'activité se situe à des températures comprises dans la gamme de 315°C à 400°C, et en ce que dans l'étage (c), le second ensemble se compose de deux lits de catalyseurs disposés en série, le lit individuel avec lequel entre tout d'abord en contact le courant de gaz dans l'étage (c) étant fait d'un catalyseur dont le maximum d'activité se situe à des températures comprises dans la gamme de 200°C à 285°C et le second lit individuel avec lequel entre en contact le courant de gaz étant fait d'un catalyseur dont le maximum d'activité se situe dans la gamme de 260c à 345°C.

5. Procédé pour la synthèse de méthanol, dans lequel on fait passer un mélange gazeux de départ, contenant de l'hydrogène et au moins l'un des oxydes de carbone, en contact de plusieurs catalyseurs successivement dans des conditions de température et de pression élevées, caractérisé par les opérations successives consistant à:

(a) faire passer le mélange gazeux de départ à travers un premier ensemble de trois lits de catalyseurs différents disposés en série, le catalyseur de chacun de ces lits ayant une composition telle qu'il ait son maximum d'activité pour la production de méthanol dans une gamme de température plus basse que le catalyseur du lit immédiatement suivant, chacun des lits étant à une température plus élevée que le précédent, le mélange de départ étant introduit dans le premier lit á une température se situant dans la gamme comprise entre 200°C et 260°C, et le mélange gazeux résultant sortant du troisième lit, aprés étre passé à travers les différents lits, à une température se situant dans la gamme comprise entre 370°C et 400°C,

(b) refroidir le courant gazeux qui sort de l'étage (a) à une température se situant dans la gamme comprise entre 200°C et 260°C, par échange de chaleur indirect,

(c) faire passer le courant gazeux qui sort de l'étage (b) à travers un second ensemble de deux lits séparés de catalyseurs différents disposés en série, le catalyseur du premier de ces lits séparés ayant une composition telle qu'il ait son maximum d'activité dans une gamme de température plus basse que le catalyseur du second des lits, le courant gazeux qui sort de l'étage (b) étant introduit dans le premier des lits du second ensemble à une température se situant dans la gamme de 200°C à 260°C et quittant le second des lits du second ensemble à une température se situant dans la gamme de 260°C à 345°C,

(d) refroidir le courant gazeux qui sort de l'étage (c) à une température se situant dans la gamme comprise entre 200°C et 260°C, par échange de chaleur indirect, et

(e) faire passer le courant gazeux qui sort de l'étage (d) à travers un lit de catalyseur final, le courant gazeux qui sort de l'étage (d) étant introduit dans ce lit final à une température se situant dans

la gamme comprise entre 200°C et 260°C et quittant ce lit final à une température qui se situe dans la gamme comprise entre 260°C et 285°C.

6. Procédé selon la revendication 5 pour le production de méthanol à partir d'hydrogène et d'oxyde de carbone, caractérisé en ce que, dans les étages (a) et (c), le lit de catalyseur avec lequel entre tout d'abord en contact le courant dans chacun du premier et du second ensemble de lits est fait de catalyseurs qui ont leur maximum d'activité à des températures se situant dans la gamme de 200°C à 285°C, en ce que, dans les étages (a) et (c), les lits suivants de catalyseurs dans le premier et le second ensembles de lits sont faits d'un catalyseur ayant son maximum d'activité à des températures se situant dans la gamme de 260°C à 335°C, et en ce que, dans l'étage (a), le troisième lit de catalyseur du premier ensemble de lits, avec lequel entre en contact le courant gazeux respectif, est fait d'un catalyseur ayant son maximum d'activité à des températures se situant dans la gamme de 315°C à 400°C.

7. Procédé selon la revendication 1, caractérisé en ce que la majorité des différents lits de catalyseurs contiennent des composés de cuivre et de zinc.

8. Procédé selon la revendication 4, caractérisé en ce que, dans les étages (a) et (c), le premier lit de chacun des premier et second ensembles de lits, avec lequel entre en contact le mélange gazeux, contient un catalyseur qui est composé d'un à six atomes de cuivre par atome de zinc.

9. Procédé selon la revendication 5, caractérisé en ce que, dans les étages (a), (c) et (e), le lit avec lequel entre tout d'abord en contact le mélange gazeux contient un catalyseur qui est composé d'un à six atomes de cuivre par atome de zinc.

10. Procédé selon la revendication 4, caractérisé en ce que, dans les étages (a) et (c), le deuxième lit avec lequel entre en contact le mélange gazeux contient un catalyseur qui est composé de 0,5 à 4 atomes de zinc par atome de cuivre.

11. Procédé selon la revendication 5, caractérisé en ce que, dans l'étage (a), le troisième lit avec lequel entre en contact le mélange gazeux contient un catalyseur qui est composé d'un à six atomes de zinc par atome de cuivre.

12. Procédé selon la revendication 1, caractérisé en ce que le catalyseur est composé d'une quantité stabilisante d'un ou de plusieurs oxydes choisis dans le groupe formé de l'aluminium, du chrome, du magnésium, du cérium, de mélanges de terres rares, de lanthane, de praséodyme, de néodyme, de zirconium, de titane, de silicium, de niobium ou de tantale ou d'un mélange de ces oxydes.

13. Procédé selon la revendication 5, caractérisé en ce que, dans l'étage (e), le catalyseur est composé de manganèse sous forme d'oxyde, en proportions suffisantes pour favoriser la production d'alcools supérieurs.

14. Procédé selon la revendication 4, caractérisé en ce que, dans les étages (a) et (c), les deux premiers lits avec lesquels entre en contact le mélange gazeux contiennent des catalyseurs qui sont composés de cuivre et de zinc, et en ce que, dans l'étage (a), le troisième lit avec lequel entre en contact le mélange gazeux contient un catalyseur qui est composé de zinc.

**Patentansprüche**

1. Verfahren zur Herstellung von Methanol, bei dem ein Wasserstoff und mindestens eines der Oxide von Kohlenstoff enthaltendes Ausgangsgasgemisch bei erhöhten Temperaturen und Drücken aufeinanderfolgend mit einer Mehrzahl von Katalysatoren in Kontakt gebracht wird, dadurch gekennzeichnet, daß man

(a) das Ausgangsgasgemisch durch eine erste Anordnung von mindestens zwei in Reihe geschalteten Betten unterschiedlicher Katalysatoren leitet, wobei der Katalysator in jedem dieser Betten eine derartige Zusammensetzung hat, daß Methanol besonders wirkungsvoll in einem niedrigeren Temperaturbereich als am Katalysator in jedem nachfolgenden Bett erzeugt wird, die jeweils in einem höheren Temperaturberich vorliegen, das Ausgangsgemisch in das erste Katalysatorbett bei einer Temperatur im Bereich von 200 bis 260°C eingeleitet wird, und das nach dem Durchtritt durch die Betten anfallende Gasgemisch das letzte Bett bei einer Temperatur im Bereich von 370 bis 400°C verläßt,

(b) daß man den in Stufe (a) anfallenden Gasstrom durch indirekten Wärmeaustausch auf eine Temperatur im Bereich von 200 bis 260°C abkühlt und

(c) daß man den in Stufe (b) erhaltenen Gasstrom durch eine zweite Anordnung von mindestens einem Bett leitet, das jeweils mit einem speziellen Katalysator beschickt ist, der besonders wirkungsvoll zur Erzeugung von Methanol innerhalb des speziellen Temperaturbereichs des darin vorherrschenden Gasstroms wirkt, wobei die Temperatur des in der Stufe (b) anfallenden Gasstroms durch die bei der Methanolbildung entstehende Reaktionswärme von 285°C auf 345°C erhöht wird.

2. Verfahren nach Anspruch 1, dadurch gekennzeichnet, daß man die Umsetzung bei einem Arbeitsdruck von 70 bis 527 kg/cm$^2$ (bar) durchführt.

3. Verfahren nach Anspruch 1, dadurch gekennzeichnet, daß das Ausgangsgasgemisch Kohlenmonoxid und Kohlendioxid enthält und zu mindestens zwei Drittel des Volumens aus Kohlenmonoxid besteht, bezogen auf das Gesamtvolumen von Kohlenmonoxid und Kohlendioxid.

4. Verfahren nach Anspruch 1 zur Herstellung von Methanol aus Wasserstoff und Kohlenmonoxid,

wobei in der Stufe (a) die erste Anordnung der Katalysatorbetten aus drei einzelnen, in Reihe geschalteten Katalysatorbetten besteht, das mit dem Ausgangsgasgemisch kontaktierte erste Bett aus einem Katalysator besteht, der besonders wirkungsvoll im Temperaturbereich von 200 bis 285°C wirkt, das zweite Bett, das mit dem Gasstrom kontaktiert wird, aus einem Katalysator besteht, der besonders wirksam bei temperaturen von 260 bis 335°C ist, das dritte, mit dem Gasstrom kontaktierte Bett aus einem Katalysator besteht, der im Temperaturbereich von 315 bis 400°C wirkt, und wobei in Stufe (c) die zweite Anordnung aus zwei, in Reihe geschalteten Katalysatorbetten besteht, wobei das mit dem Gasstrom in Stufe (c) zunächst kontaktierte Bett aus einem Katalysator besteht, der besonders wirkungsvoll im Temperaturbereich von 200 bis 285°C wirkt und das zweite, mit dem Gasstrom kontaktierte Bett aus einem Katalysator besteht, der besonders wirkungsvoll im Temperaturbereich von 260 bis 234°C wirkt.

5. Verfahren zur Herstellung von Methanol, wobei ein Wasserstoff und mindestens eines der Oxide von Kohlenstoff enthaltendes Ausgangsgasgemisch nacheinander in Kontakt mit einer Mehrzahl von Katalysatoren bei erhöhten Temperaturen und Drücken gebracht wird, dadurch gekennzeichnet, daß man

(a) das Ausgangsgasgemisch durch eine erste Anordnung von drei in Reihe geschalteten Betten verschiedener Katalysatoren leitet, wobei der Katalysator in jedem der Betten eine derartige Zusammensetzung hat, daß er Methanol besonders wirkungsvoll in einem niedrigeren Temperaturbereich bildet als der Katalysator in jedem nachfolgenden Bett, wobei diese Betten in einem höheren Temperaturbereich vorliegen, das Ausgangsgasgemisch in das erste Katalysatorbett bei einer Temperatur im Bereich von 200 bis 260°C eingeleitet wird, und das nach dem Durchtritt durch die Betten anfallende Gasgemisch das dritte Bett bei einer Temperatur im Bereich von 370 bis 400°C verläßt,

(b) daß man den in Stufe (a) anfallenden Gasstrom durch indirekten Wärmeaustausch auf eine Temperatur im Bereich von 200 bis 260°C abkühlt,

(c) daß man den in der Stufe (b) anfallenden Gasstrom durch eine zweite Anordnung aus zwei einzelnen, in Reihe geschalteten Betten verschiedener Katalysatoren leitet, wobei der Katalysator in dem ersten Bett eine derartige Zusammensetzung hat, daß er besonders wirkungsvoll in einem niedrigeren Temperaturbereich als der Katalysator in dem zweiten Bett wirkt, der in Stufe (b) anfallende Gasstrom in das erste Bett dieser zweiten Anordnung bei einer Temperatur von 200 bis 260°C eingeleitet und aus dem zweiten Bett dieser zweiten Anordnung bei einer Temperatur im Bereich von 260 bis 345°C austritt,

(d) daß man den in Stufe (c) erhaltenen Gasstrom durch indirekten Wärmeaustausch auf 200 bis 260°C abkühlt und

(e) daß man den in Stufe (d) erhaltenen Gasstrom durch ein letztes Katalysatorbett leitet, wobei der in Stufe (d) anfallende Gasstrom in dieses letzte Katalysatorbett bei einer Temperatur von 200 bis 260°C eingeleitet wird und der Gasstrom aus dem letzten Katalysatorbett bei einer Temperatur von 260 bis 285°C auftritt.

6. Verfahren nach Anspruch 5 zur Herstellung von Methanol aus Wasserstoff und Kohlenmonoxid, dadurch gekennzeichnet, daß in den Stufen (a) und (c) das anfänglich mit dem Gasstrom kontaktierte Katalysatorbett in jeder der ersten und zweiten Bettenanordnung aus Katalysatoren besteht, die besonders wirkungsvoll im Temperaturbereich von 200 bis 285°C wirken, wobei in den Stufen (a) und (c) die nächsten Katalysatorbetten in der ersten und zweiten Bettenanordnung aus einem Katalysator bestehen, der besonders wirkungsvoll bei Temperaturen im Bereich von 260 bis 335°C wirkt, und wobei in Stufe (a) das dritte Katalysatorbett in der ersten Anordnung, das mit dem jeweiligen Gasstrom kontaktiert wird, aus einem Katalysator besteht, der besonders wirkungsvoll bei Temperaturen von 315 bis 400°C wirkt.

7. Verfahren nach Anspruch 1, dadurch gekennzeichnet, daß die Mehrzahl der einzelnen Katalysatorbetten Kupfer- und Zink-Verbindungen enthält.

8. Verfahren nach Anspruch 4, dadurch gekennzeichnet, daß in den Stufen (a) und (c) das erste Bett der ersten und zweiten Anordnung, das mit dem Gasgemisch kontaktiert wird, einen Katalysator enthält, der 1 bis 6 Kupferatome pro Atom Zink aufweist.

9. Verfahren nach Anspruch 5, dadurch gekennzeichnet, daß in den Stufen (a), (c) und (e) das anfänglich mit dem Gasgemisch kontaktierte Bett einen Katalysator enthält, der 1 bis 6 Atome Kupfer pro Atom Zink enthält.

10. Verfahren nach Anspruch 4, dadurch gekennzeichnet, daß in den Stufen (a) und (c) das zweite, mit dem Gasgemisch kontaktierte Bett einen Katalysator enthält, der 0,5 bis 4 Atome Zink pro Atom Kupfer enthält.

11. Verfahren nach Anspruch 5, dadurch gekennzeichnet, daß in Stufe (a) das dritte mit dem Gasgemisch kontaktierte Bett einen Katalysator enthält, der aus 1 bis 6 Atomen Zink pro Atom Kupfer besteht.

12. Verfahren nach Anspruch 1, dadurch gekennzeichnet, daß der Katalysator eine stabilisierende Menge eines oder mehrerer Oxide der Gruppe Aluminium, Chrom, Magnesium, Cer, Seltene Erdengemische, Lanthan, Praseodym, Neodym, Zirkonium, Titan, Silicium, Niob oder Tantal oder deren Gemisch enthält.

13. Verfahren nach Anspruch 5, dadurch gekennzeichnet, daß in Stufe (e) der Katalysator Mangan als Oxid in einer Menge enthält, die zur Förderung der Bildung höherer Alkohole ausreicht.

14. Verfahren nach Anspruch 4, dadurch gekennzeichnet, daß in den Stufen (a) und (c) die ersten, mit dem Gasgemisch kontaktierten beiden Betten Katalysatoren enthalten, die aus Kupfer und Zink bestehen und wobei in Stufe (a) das dritte, mit dem Gasgemisch Kontaktierte Bett einen Zink enthaltenden Katalysator enthält.

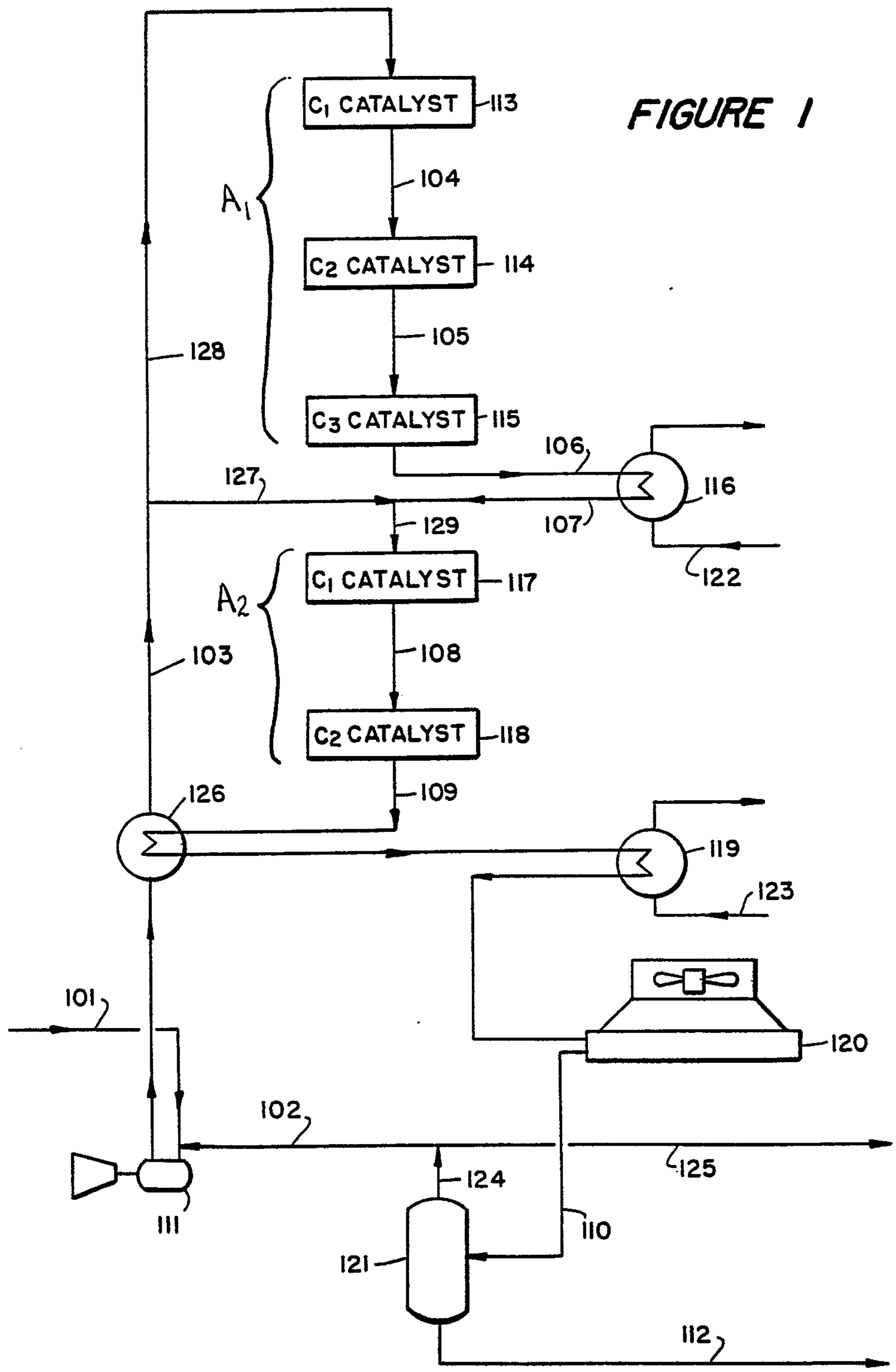

C1 CATALYST
113
104
A1
C2 CATALYST
114
105
128
C3 CATALYST
115
106
116
127
107
129
122
C1 CATALYST
117
A2
108
103
C2 CATALYST
118
109
126
119
123
120
101
102
125
124
111
110
121
112


FIGURE 1

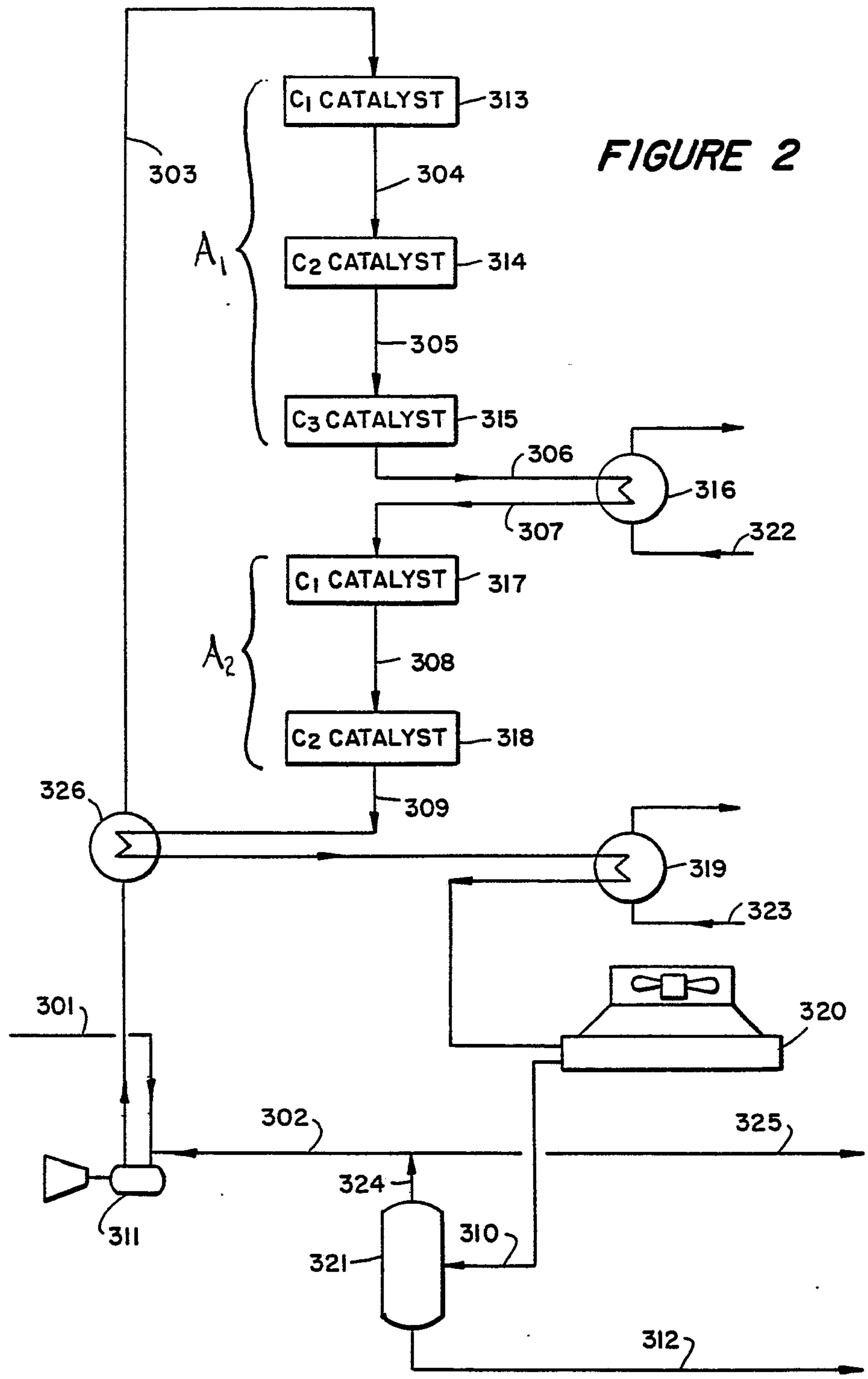
C1 CATALYST
313
303
304
A1
C2 CATALYST
314
305
C3 CATALYST
315
306
316
307
322
C1 CATALYST
317
A2
308
C2 CATALYST
318
326
309
319
323
320
301
302
325
324
311
310
321
312

FIGURE 2

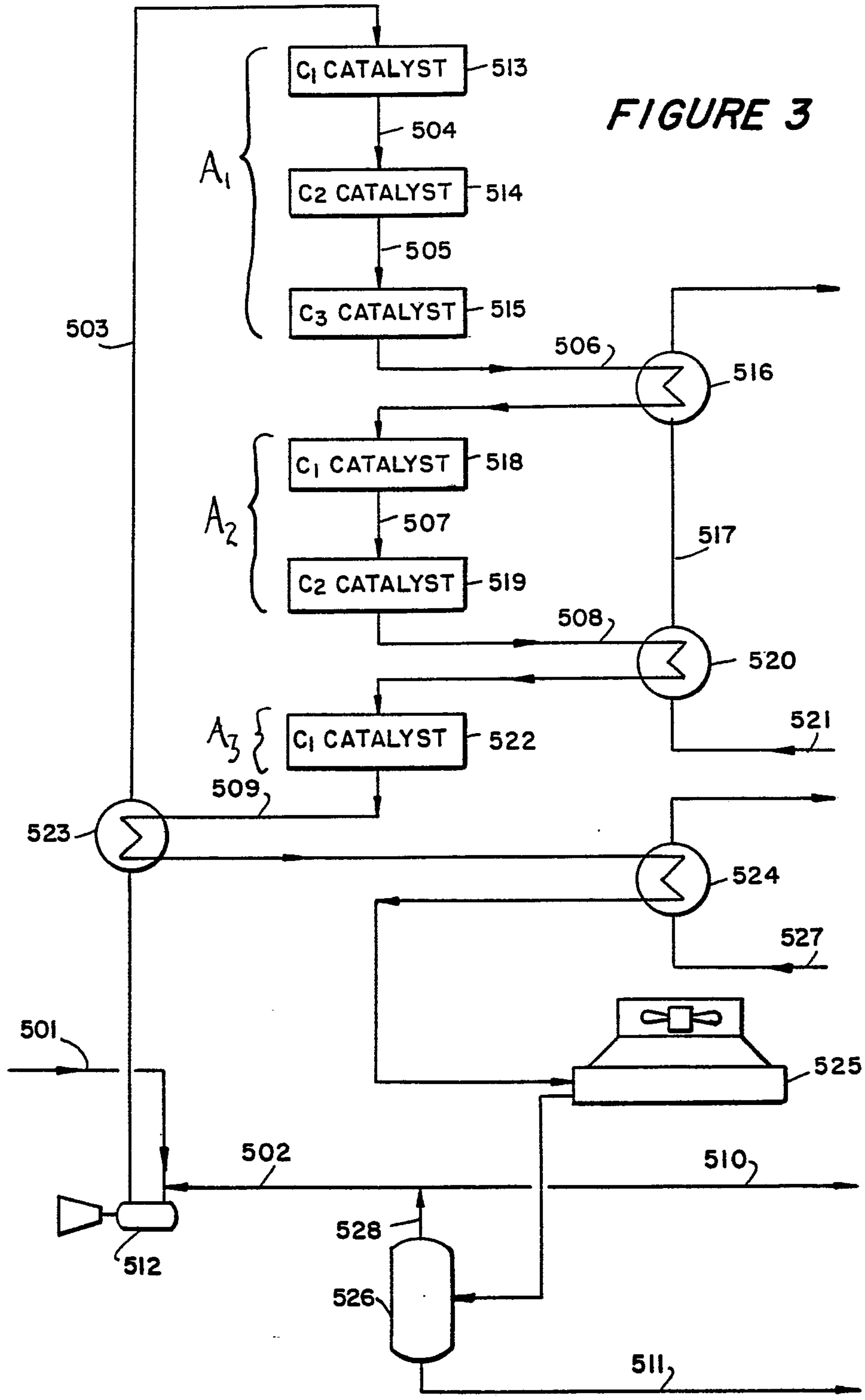
FIGURE 3
C1 CATALYST
513
504
C2 CATALYST
514
505
C3 CATALYST
515
A1
503
506
516
C1 CATALYST
518
507
C2 CATALYST
519
A2
517
508
520
A3
C1 CATALYST
522
521
509
523
524
527
501
525
502
510
528
512
526
511

FIGURE 4

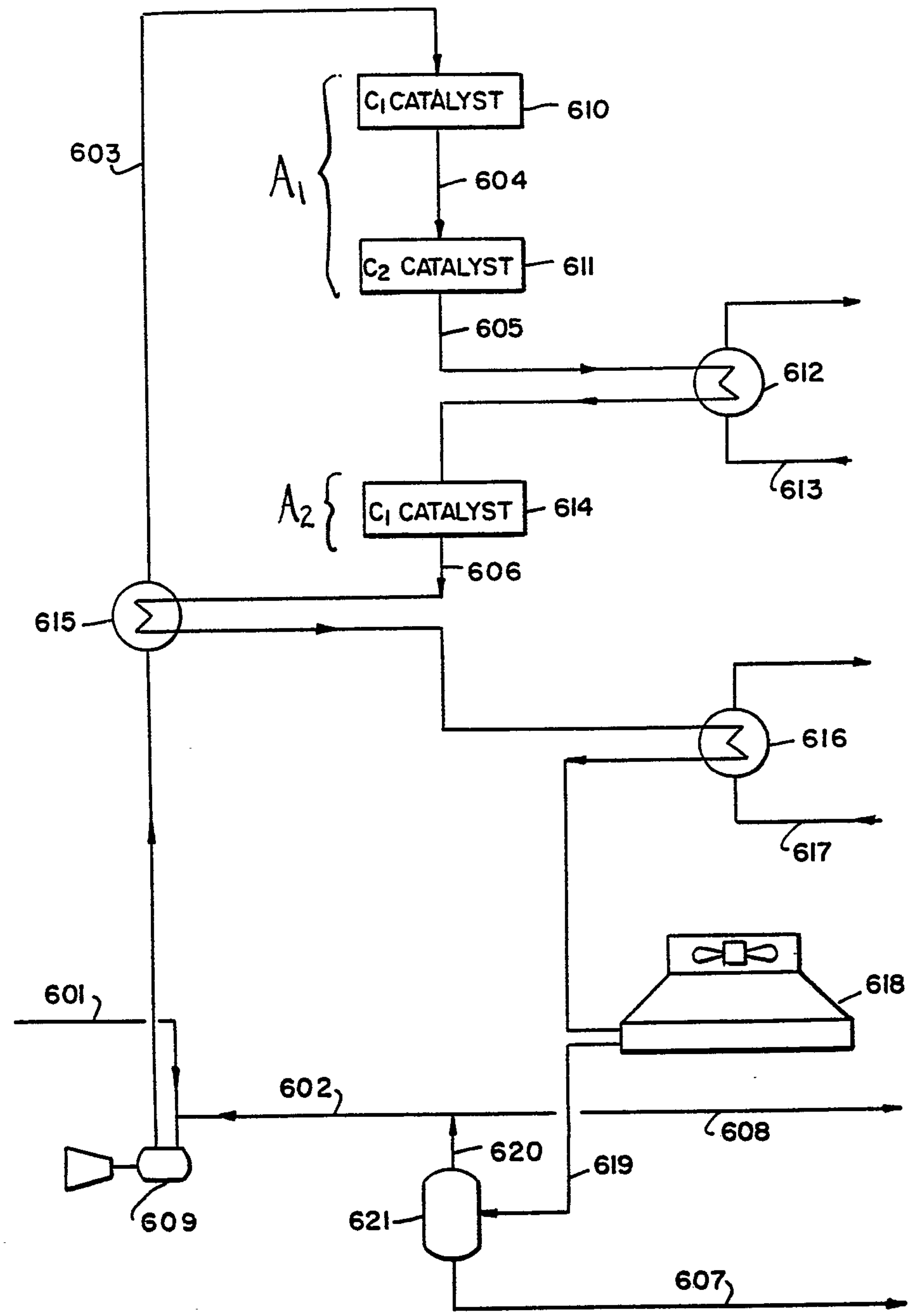

C1 CATALYST
610
603
A1
604
C2 CATALYST
611
605
612
613
A2
C1 CATALYST
614
606
615
616
617
618
601
602
608
620
619
609
621
607

FIGURE 5

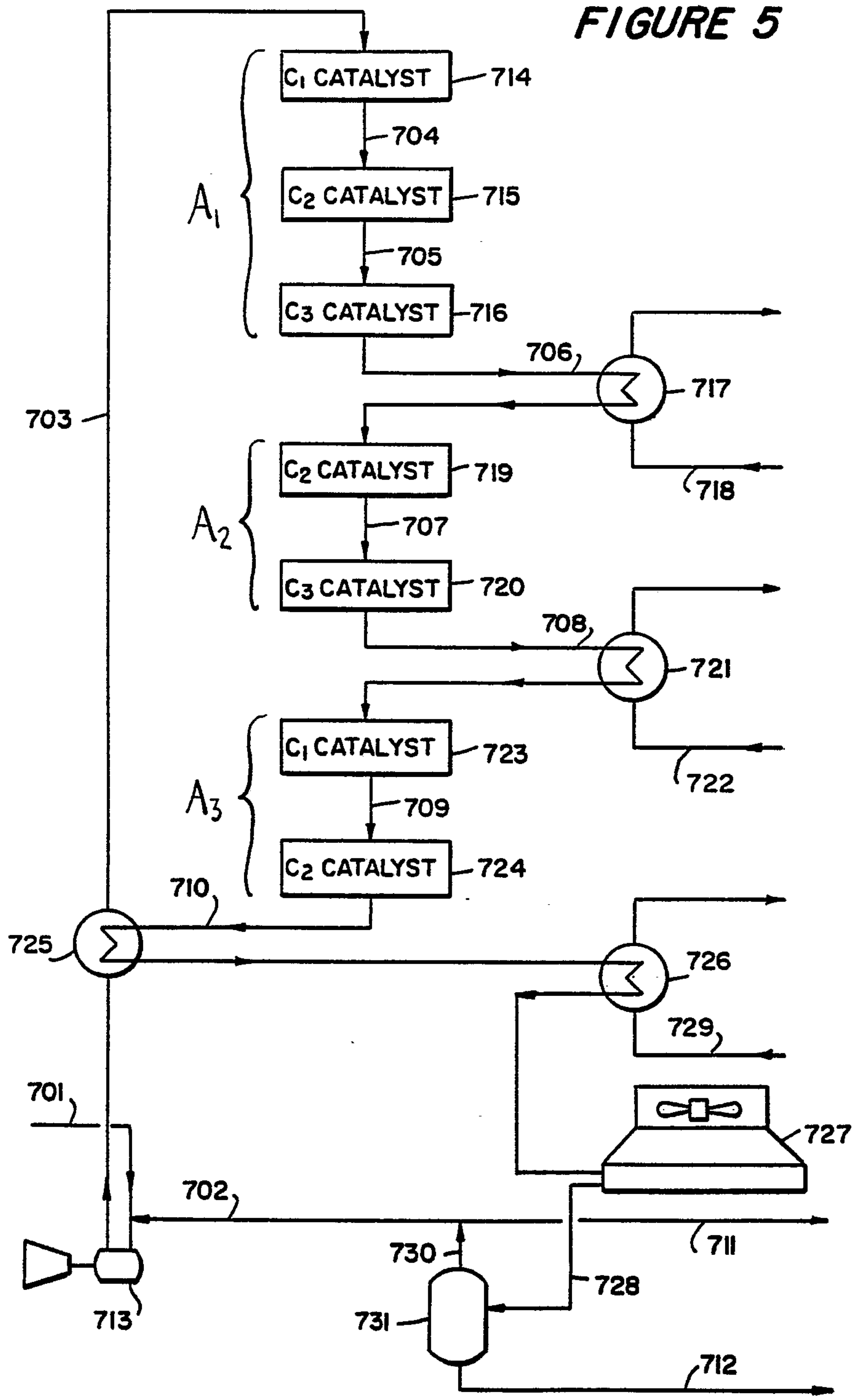

C1 CATALYST
714
704
C2 CATALYST
715
705
C3 CATALYST
716
A1
706
717
703
C2 CATALYST
719
718
A2
707
C3 CATALYST
720
708
721
C1 CATALYST
723
722
A3
709
C2 CATALYST
724
710
725
726
729
701
727
702
711
730
728
731
713
712